# EUROPÄISCHE PATENTSCHRIFT

(11) **EP 1 292 250 B1**
(45) Veröffentlichungstag und Bekanntmachung des Hinweises auf die Patenterteilung: **26.01.2005**
(21) Anmeldenummer: 00934834.3
(22) Anmeldetag: 19.06.2000
(51) Int. Cl.: A61F 2/38

(54) **KNIEGELENKENDOPROTHESE**
KNEE-JOINT ENDOPROSTHESIS
ENDOPROTHESE DU GENOU

(43) Veröffentlichungstag der Anmeldung: 19.03.2003
(73) Patentinhaber: Mathys Medizinaltechnik AG, 2544 Bettlach (CH)
(72) Erfinder: WEHRLI, Ulrich, CH-3084 Wabern (CH); SUPPER, Walter, CH-2544 Bettlach (CH)
(74) Vertreter: Lusuardi, Werther Giovanni, Dr.
(86) Internationale Anmeldenummer: PCT/CH2000/000330
(87) Internationale Veröffentlichungsnummer: WO 2001/097719

(56) Entgegenhaltungen:
- EP-A- 0 529 408
- DE-A- 4 308 563
- FR-A- 2 771 281
- US-A- 5 702 466

## Beschreibung

Die Erfindung betrifft eine Kniegelenkendoprothese mit einem Tibiateil, bestehend aus einem Verankerungsteil und einer Tiblalagerfläche, einem auf dieser verschiebbaren Lagerkörper mit zwei konkav gekrümmten Lagerschalen nach dem Oberbegriff des Anspruchs 1 und einem Verfahren zur Herstellung einer solchen Kniegelenkendoprothese nach dem Oberbegriff des Anspruchs 18.

Um die komplizierte Dreh- und Gleitbewegung eines Kniegelenks bei einer Kniegelenkendoprothese soweit wie möglich nachzuahmen, ist es bekannt, auf einer ebenen Tibialagerfläche einen Lagerkörper frei verschieblich zu lagern, auf dem ein Femurgelenk verschieblich aufliegt.

Bei einfachen Ausgestaltungen ist der Lagerkörper der ebenen Tiblalagerfläche verschieblich und wird nur durch die die beiden Gelenkteile festhaltenden Bänder in seiner Verschiebebewegung begrenzt (GB-PS 1 603 833), bei anderen Ausgestaltungen begrenzt ein aus der Tibialagerfläche nach oben vorstehender Vorsprung, der in eine Ausnehmung an der Unterseite des Lagerkörpers eingreift, diese Bewegung (US-PS 4 085 466). Es ist weiterhin bekannt, den Lagerkörper zu teilen und die dabei entstehenden Lagerschalen auf der Tiblalagerfläche längs einer gekrümmten Führung zu führen, die bspw. im Querschnitt schwalbenschwanzförmig ausgebildet sein kann (EP-B1-0 021 421). Diese zuletzt genannte Lagerung ermöglicht zwar eine exakte Führung der beiden Lagerkörperteile längs dieser Führung, die Konstruktion ist jedoch infolge der beiden Längsführungen relativ kompliziert und schränkt insbesondere die Drehbewegung des gesamten Gelenkes ein.

Es sind daneben auch Kniegelenkendoprothesen bekannt, die nur eine Rotation des Lagerkörpers relativ zur Tiblalagerfläche ermöglichen, dagegen keine Transversalverschiebung. Bei dieser Kniegelenkendoprothese wird zwar ein relativ unkomplizierter Aufbau erreicht, da das Kniegelenk nur eine Rotation des Lagerkörpers zuläßt, doch kann die natürliche Bewegung nur teilweise nachgebildet werden.

In der EP 186 471 A3 ist eine Kniegelenkendoprothese beschrieben, bei welcher ein Lagerkörper auf der Tiblalagerfläche um eine senkrecht auf dieser stehenden Drehachse geführt wird. Diese Drehachse wird durch einen Zapfen gebildet, der seinerseits in einer Kunststoffhülse geführt wird. Der Lagerkörper kann bei dieser Prothese nur eine Rotationsbewegung ausführen.

Aus der EP 0 519 873 B1 ist eine Kniegelenkendoprothese bekannt, die für die Tiblalagerfläche einen Verankerungsabschnitt und für die Kondylenplatte einen Drehgelenkabschnitt aufweist. Es ist ein Kupplungsorgan vorgesehen, das mittels eines Führungsteils und einer nicht als Langloch ausgebildeten Führungsbahn, welche das Führungsteil aufnimmt und in welcher dieses medial und lateral geführt gleiten kann, zusammenwirkt.

Die einzelnen Komponenten lassen diese Kniegelenkendoprothese kostenungünstig werden, sie ist störanfällig und verschleiß-behaftet.

Aus der EP 0529408B1 ist eine Kniegelenkendoprothese bekannt, die das Problem der Rotation aufgreift und einen auf der ebenen Tiblalagerfläche angeordneten, an dieser um eine senkrecht zur Tibialagerfläche angeordnete Drehachse drehbar gelagerten geradlinigen stabförmigen Lenker umfaßt, der zwei in paralleler Richtung verlaufende, seitliche Führungsflächen aufweist, und wobei der Lagerkörper an seiner Unterseite eine den stabförmigen Lenker aufnehmende nach unten offene Nut trägt, deren Seitenwände in paralleler Richtung verlaufende Führungsflächen des Lagerkörpers bilden, an denen die seitliche Führungsfläche des Lenkers anliegen und diesen bei einer parallel zum Lenker erfolgenden freien Verschiebung führen. Der Lenker ermöglicht zwar die freie Verschiebbarkeit des Lagerkörpers längs des Lenkers, so daß eine überlagerte Drehverschiebungsbewegung möglich wird, wobei dies in einfacher Weise durch den Lenker erzielbar ist, wobei allerdings der Lenkerkörper nicht in den Lagerkörper eingerastet ist, woraus eine geringe mechanische Stabilität resultiert.

Hier gilt es Abhilfe zu schaffen. Der vorliegenden Erfindung liegt die Aufgabe zugrunde, eine Kniegelenkendoprothese anzugeben, die mechanisch stabiler ist und die unterschiedlichen Bewegungsabläufe der Kniegelenkendoprothese quasi selbsttätig sich überlassend ausführt und bei der das Dynamikverhalten verbessert ist.

Diese Aufgabe wird erfindungsgemäß durch den kennzeichnenden Teil des Anspruchs 1 gelöst.

Weitere vorteilhafte Ausgestaltungen der Erfindung ergeben sich aus den Unteransprüchen.

Die erfindungsgemäße Kniegelenkendoprothese hat den Vorteil, daß der Lenkerkörper mittels der Schnappverbindung ab Werk vormontiert ist und die Operation hierdurch einfacher gestaltet werden kann.

Bei einer bevorzugten Ausführungsform besteht der zusätzliche Vorteil, dass sich durch die Hydrodämpfung mittels des Lenkerkörpers in der Sacklochbohrung und der Synovia, die durch die kleine Öffnung der Sacklochbohrung am anterioren Ende eindringen kann, der dynamische Bewegungsablauf natürlicher gestalten lässt.

In einer vorteilhaften Ausgestaltung ist der Querschnitt des Lenkerkörpers zur Erzeugung einer Schnappverbindung kreiszylinderförmig ausgestaltet. Dies hat den Vorteil, dass die Krafteinleitung immer zentriert erfolgt.

Weiterhin ist vorgesehen, den Hintergriff des zylinderförmigen Lenkerteils durch den Hintergriff der Schnappverbindung in einer entsprechenden Ausnehmung oder Sacklochbohrung gegen Endanschläge in anteriorer bzw. posteriorer Richtung steuerbar und begrenzbar zu machen.

Ein Ausführungsbeispiel der Erfindung ist in den Figuren dargestellt.

Es zeigen:
- Fig. 1:: eine perspektivische Ansicht einer Kniegelenkendoprothese,
- Fig. 2:: einen perspektivischen Schnitt durch eine Kniegelenkendoprothese,
- Fig. 3:: einen Schnitt durch die Achse 1/1,
- Fig. 4:: einen Schnitt durch die Schnappverbindung.

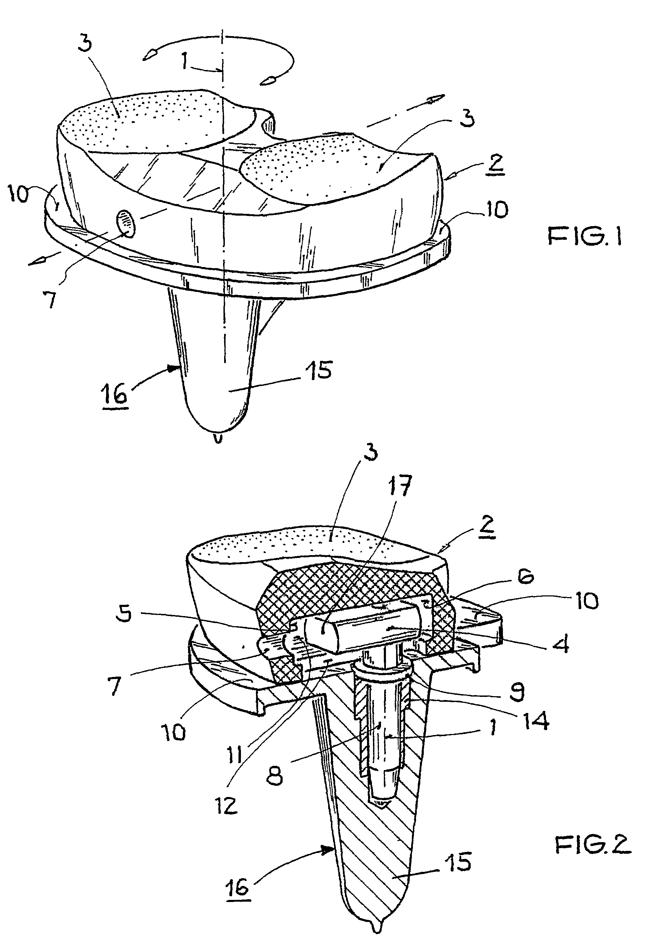

Die in Fig. 1 dargestellte Kniegelenkendoprothese in perspektivischer Ansicht zeigt das Tiblateil 16, das die Tiblalagerfläche 10 und das Verankerungsteil 15, wobei das Verankerungsteil 15 auf eine entsprechend ausgestaltete Tibiaoberseite aufsetzbar und in dieser verankerbar ist. Auf der Tiblaoberfläche ist die Kondylenplatte 2 angeordnet, die aus Kunststoff, wie beispielsweise Polyethylen besteht. Die Kondylenplatte 2 weist zwei nebeneinanderliegende Lagerschalen 3 für die Femurkondylen auf. Diese Lagerschalen 3 sind über eine Brücke miteinander verbunden, so daß die Kondylenplatte 2 insgesamt einstückig ausgebildet ist.

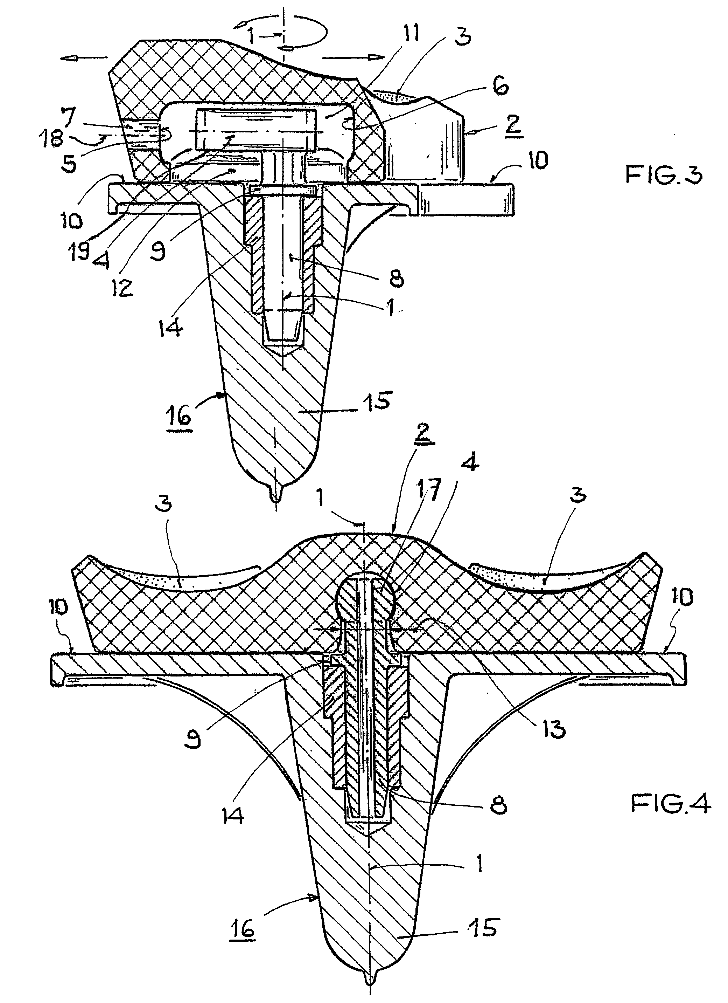

Wie eine Zusammenschau der Fig. 1, Fig. 2 und Fig. 3 zeigt, ist der Lagerkörper bzw. die Kondylenplatte 2 mit ihrer ebenen Unterseite flächig aufgelegt und gegenüber der Tibialagerfläche 10 sowohl in translatorischer als auch rotatorischer Richtung frei verschieblich. Die Kondylenplatte 2 wird zu diesem Zweck senkrecht zu einer auf der Tibialagerfläche 10 stehenden Drehachse 1 mittels des Lenkerkörpers 4 geführt. Der Lenkerkörper 4 weist eine kreiszylinderförmige Gestalt auf, wobei der Lenkerkörper 4 sich in Richtung seiner Längsachse 18 längs erstreckt und stabförmig ausgebildet ist und aus der Ebene der Tiblalagerfläche 10 herausragt. Zwischen der Unterseite 19 (Fig.3) des Lenkerkörpers 4 und der Tibialagerfläche 10 ist ein definierter Abstand von mindestens 3 mm, vorzugsweise mindestens 4 mm vorhanden. An seinem rechten Ende (Fig. 3) weist der Lenkerkörper den Lagerzapfen 8 aufweist, der im Verankerungsteil 15 der Tibialagerfläche 10 drehbar gelagert ist. Der Lagerkörper 4 wirkt mit der Kondylenplatte 2 zusammen, indem in der Kondylenplatte 2 die Sacklochbohrung 11 vorgesehen ist mit der länglichen Öffnung 12, die mindestens so lang ist wie die Längserstreckung des Lenkerkörpers 4 und in der Breite 13 schmäler als der Durchmesser des kreissegmentförmigen Querschnitts 17 des Lenkerkörpers 4. Zwischen Lenkerkörper 4 einerseits und Sacklochbohrung 11 mit länglicher Öffnung 12 entsteht dadurch eine Schnappverbindung, so daß die Kondylenplatte 2 längs der Innenfläche der Sacklochbohrung 11 am Lenkerkörper 4 in anteriorer bzw. posteriorer Richtung entlanggleiten kann. Der Lenkerkörper 4 wirkt im Zusammenspiel mit seinem Lagerzapfen 8, der durch den Dichtring-Anschlag 9 abgedichtet ist, so daß translatorische als auch rotatorische Bewegungen der Kondylenplatte 2 möglich sind.

Der Lenkerkörper 4 weist den nach unten abstehenden Lagerzapfen 8 auf, der im Verankerungsteil 15 verankert ist. Die Kondylenplatte 2 ist mittels Lenkerkörper 4 und Lagerzapfen 8 um die Drehachse 1 schwenkbar. Daraus ergibt sich eine komplexe Schwenk- und Translationsbewegung, die für alle in der Praxis auftretenden Bewegungen ausreichend ist und die eine volle Nachbildung der natürlichen Beweglichkeit einer Kniegelenkendoprothese ermöglicht.

Mittels der kleinen Öffnung 7, deren Durchmesser kleiner ist als der Durchmesser des kreissegmentförmigen Querschnitts des Lenkerkörpers 4 an der Stirnseite der Sacklochbohrung 11 am anterioren Ende der Kondylenplatte 2 gelangt Synovia in die Sacklochbohrung 11 und diese wirkt zusammen mit dem bezüglich des Querschnitts kreissegmentförmigen Lenkerkörper 4 und der Synovia als Hydrodämpfer. Der Lagerzapfen 8 weist mittels der in das Verankerungsteil 15 eingepreßten Metallhülse 14 eine Metall-Metallführung auf, die verschleißfester ist als vergleichbare Konstruktionen bei herkömmlichen Kniegelenkendoprothesen.

Die Kondylenplatte 2 ist vorzugsweise maximal um die Länge der Sacklochbohrung 11 minus der Länge des Lenkerkörpers 4 in anteriorer bzw. posteriorer Richtung verschiebbar. Je nach Auslegung der länglichen Öffnung 12, der Länge der Sacklochbohrung 11 und der Länge des Lenkerkörpers 4 ist auch eine Längsbewegung der Kondylenplatte 2 zwischen den Endanschlägen 5 und 6 der Sacklochbohrung 11 möglich. Wesentlich ist, daß auf diese Art und Weise die Transversalbewegung der Kondylenplatte 2 begrenzt ist (vorzugsweise im Bereich von 6 bis 13 mm, typischerweise - je nach Grösse der Endogelenkprothese - im Bereich von 7,5 mm bis 11,5 mm).

Der Lenkerkörper 4 wird vorteilhafterweise durch eine Vormontage in die Sacklochbohrung 11 mit Schnappverbindung eingepreßt.

Die Tibialagerfläche 10, auf der die Kondylenplatte 2 drehbar ist, wird mittels des Verankerungsteils 15 im Knochen befestigt und kann sowohl zementiert oder zementfrei befestigt sein. Die Translations- und Rotationsbewegung der Kondylenplatte 2 auf der Tiblalagerfläche 10 bilden die Voraussetzung für einen möglichst naturgetreuen Bewegungsablauf des Kniegelenks.

In die kleine Öffnung 7 fließt sogenannte Synovia (von der Synovalis gebildete Gelenkschmiere), eine fadenziehende Flüssigkeit, die Fetttröpfchen, Eiweiss, Hyaluronsäure und Zelltrümmer enthält. Es erfolgt keine Abdichtung der Sacklochbohrung 11 mit der kleinen Öffnung 7, da sie bei der Kondylenplatte 2 in anteriorer Richtung geöffnet ist. Die Synovia kann somit durch diese kleine Öffnung 7 in die Sacklochbohrung 11 eindringen. Da die kleine Öffnung 7 im Querschnitt kleiner ist als der Durchmesser des Querschnitts der Sacklochbohrung, kann dies als eine Art Dämpfer bezeichnet werden. Die sich in der Sacklochbohrung 11 befindende Flüssigkeit wird bei anterior-posterioren Bewegungen aus der Sacklochbohrung 11 durch die anteriore kleine Öffnung 7 bei der Kondylenplatte 2 gedrückt.

Gegenüber dem bekannten und angezogenen Stand der Technik wird bei dieser Erfindung die Anterior-Posterior-Bewegung der Kondylenplatte 2 begrenzt und der Lenkerkörper 4 wird mittels Schnappverbindung vormontiert.

## Patentansprüche

1. Kniegelenkendoprothese mit einem Tiblateil (16), bestehend aus einem in der Tibia fixierbaren Verankerungsteil (15) und einer Tibialagerfläche (10), einer auf dieser verschiebbar angeordneten Kondylenplatte (2) mit zwei konkav gekrümmten Lagerschalen (3) zur beweglichen Aufnahme eines Femurgelenkteils und mit Mitteln (2,4,10) zu einer Drehführung der Kondylenplatte (2) sowohl in rotatorischer als auch transversaler Richtung bezüglich der Tibialagerfläche (10), wobei die Kondylenplatte (2) auf der Tiblalagerfläche (10) um eine senkrechte auf dieser stehenden Drehachse (1) verdrehbar und längsverschiebbar ist und wobei die Mittel (2,4,10) zur Drehführung einen auf der ebenen Tibialagerfläche (10) angeordneten an dieser um eine senkrecht zur Tibialagerfläche (10) angeordneten Drehachse (1) drehbar gelagerten, geradlinigen stabförmigen Lenkerkörper (4) umfassen,
**dadurch gekennzeichnet, dass**
der Lenkerkörper (4) zylinderförmig ausgestaltet ist und in einem definierten Abstand von der Tibialagerfläche (10) positioniert ist und einen Lagerzapfen (8) aufweist, der im Verankerungsteil (15) des Tibiateils (16) verdrehbar gelagert ist, wobei der Lenkerkörper (4) mit der Kondylenplatte (2) zusammenwirkt, indem in der Kondylenplatte (2) eine Sacklochbohrung (11) vorgesehen ist mit einer länglichen Öffnung (12), die mindestens so lang ist wie die Längserstreckung des Lenkerkörpers (4) und in der Breite (13) schmäler ist als der Durchmesser der Querschnittsfläche des Lenkerkörpers (4), so daß zwischen Lenkerkörper (4) einerseits und Sacklochbohrung (11) mit länglicher Öffnung andererseits eine Schnappverbindung entsteht und die Kondylenplatte (2) in Richtung der Längsachse (18) des Lenkerkörpers (4) gleitbar und um die Drehachse (1) verdrehbar ist.

2. Kniegelenkendoprothese nach Anspruch 1, **dadurch gekennzeichnet, daß** der Lenkerkörper (4) kreiszylinderförmig ausgestaltet ist.

3. Kniegelenkendoprothese nach Anspruch 1 oder 2, **dadurch gekennzeichnet, daß** der Lenkerkörper (4) an seiner der Tibialagerfläche (10) gegenüberliegenden Seite abgeflacht ist.

4. Kniegelenkendoprothese nach Anspruch 1 oder 2, **dadurch gekennzeichnet, daß** der Lenkerkörper (4) an seiner den Lagerschalen (3) gegenüberliegenden Seite abgeflacht ist.

5. Kniegelenkendoprothese nach einem der Ansprüche 1 bis 4, **dadurch gekennzeichnet, daß** der Lenkerkörper (4) tonnenförmig ausgebildet ist.

6. Kniegelenkendoprothese nach einem der Ansprüche 1 bis 5, **dadurch gekennzeichnet, daß** die seitlichen Bereiche der Aussenfläche des Lenkerkörper (4) eine gekrümmte Fläche, vorzugsweise mit einer gemeinsamen Krümmungsachse, aufweisen.

7. Kniegelenkendoprothese nach Anspruch 6, **dadurch gekennzeichnet, dass** die Krümmungsachse mit der Längsachse (18) des Lenkerkörpers (4) zusammenfällt.

8. Kniegelenkendoprothese nach einem der Ansprüche 1 bis 7, **dadurch gekennzeichnet, daß** die zur Längsachse (18) des Lenkerkörpers (4) verlaufenden Aussenflächen keine zueinander parallele Flächen aufweisen.

9. Kniegelenkendoprothese nach einem der Ansprüche 1 bis 8, **dadurch gekennzeichnet, daß** der Querschnitt (17) des Lenkerkörper (4) kreisförmig, vorzugsweise kreissegmentförmig ausgebildet ist,

10. Kniegelenkendoprothese nach einem der Ansprüche 1 bis 9, **dadurch gekennzeichnet, daß** die Sacklochbohrung (11) mit ihrem Querschnitt so ausgebildet ist, daß mittels einer Öffnung (7), die im Durchmesser kleiner ist als der Durchmesser der Sacklochbohrung (11), an der Stirnseite der Sacklochbohrung (11) am anterioren Ende der Kondylenplatte (2) Synovia eindringbar ist und Sacklochbohrung (11) und Lenkerkörper (4) im Zusammenwirken mit der Synovia als Hydrodämpfer wirken.

11. Kniegelenkendoprothese nach einem der Ansprüche 1 bis 10, **dadurch gekennzeichnet, daß** sowohl der Lagerzapfen (8) des Lenkerkörpers (4) als auch der Verankerungsteil (15) der Tibialagerfläche (10) aus einem metallischen Werkstoff, vorzugsweise aus dem gleichen metallischen Werkstoff gefertigt sind, so dass eine reine Metall-Metall-Führung des Lagerzapfens (8) resultiert.

12. Kniegelenkendoprothese nach einem der Ansprüche 1 bis 10, **dadurch gekennzeichnet, daß** der Verankerungsteil (15) der Tibialagerfläche (10) mit einer vorzugsweise darin eingepressten Metallhülse (14) versehen ist, welche zur Aufnahme des Lagerzapfens (8) bestimmt ist und dass der Lagerzapfen (8) ebenfalls aus einem metallischen Werkstoff, vorzugsweise aus dem gleichen metallischen Werkstoff wie die Metallhülse (14) gefertigt ist, so dass eine reine Metall-Metallführung resultiert.

13. Kniegelenkendoprothese nach einem der Ansprüche 1 bis 12, **dadurch gekennzeichnet, daß** die Kondylenplatte (2) maximal um die Länge der Sackbohrung (11) minus der Länge des Lenkerkörpers (4) in anteriorer bzw. posteriorer Richtung verschiebbar ist.

14. Kniegelenkendoprothese nach einem der Ansprüche 1 bis 13, **dadurch gekennzeichnet, daß** die Kondylenplatte (2) um den Betrag 6 mm < x < 13 mm, vorzugsweise 7,5 mm < x < 11,5 mm in anteriorer bzw. posteriorer Richtung verschiebbar ist.

15. Kniegelenkendoprothese nach einem der Ansprüche 1 bis 14, **dadurch gekennzeichnet, daß** die Länge der Sackbohrung (11) um 0,1 bis 3,0 mm, vorzugsweise um 1 bis 2 mm länger ist, als die Länge des Lenkerkörpers (4).

16. Kniegelenkendoprothese nach einem der Ansprüche 1 bis 15, **dadurch gekennzeichnet, dass** zwischen der zur Längsachse (18) des Lenkerkörper (4) verlaufenden Aussenfläche des Lenkerkörpers (4) und der damit korrespondierenden Innenfläche der Sacklochbohrung (11) ein Spiel von 0,1 bis 0,4 mm, vorzugsweise von 0,2 bis 0,3 mm vorgesehen ist.

17. Kniegelenkendoprothese nach einem der Ansprüche 1 bis 16, **dadurch gekennzeichnet, dass** die Unterseite (19) des Lenkerkörpers (4) einen Abstand von mindestens 3 mm, vorzugsweise von mindestens 4 mm von der Ebene der Tibialagerfläche (10) aufweist.

18. Verfahren zur Herstellung einer Kniegelenkendoprothese nach einem der Ansprüche 1 bis 17, **dadurch gekennzeichnet, daß** der Lenkerkörper (4) durch eine Vormontage in die Sacklochbohrung (11) mit der Schnappverbindung eingepreßt wird.

## Claims

1. A knee joint endoprosthesis with a tibial component (16), comprising an anchoring part (15) that can be fastened in the tibia and a tibial support surface (10), a condylar plate (2) provided on it in a displaceable manner with two concavely curved support shells (3) to displaceably accommodate the femur and with means (2, 4, 10) to rotationally guide the condylar plate (2) both in the rotational and transversal directions relative to the tibial support surface (10), whereby the condylar plate (2) can rotate and be longitudinally displaced on the tibial support surface (10) about an axis of rotation (1) that is perpendicular to the tibial support surface and whereby the means (2, 4, 10) for the rotational guiding comprise a straight rod-shaped guide body (4) rotatably mounted about an axis of rotation (1) arranged on the flat tibial support surface (10) perpendicularly to the tibial support surface (10),
**characterized in that**
the guide body (4) has a cylindrical construction and is positioned at a defined distance form the tibial support surface (10) and has a bearing spigot (8) that is rotatably mounted in the anchoring part (15) of the tibial component (16), whereby the guide body (4) interacts with the condylar plate (2) by virtue of a blind hole (11) with a longish opening (12) being provided in the condylar plate (2), the blind hole being at least as long as the length of the guide body (4) and its width (13) being narrower than the diameter of the circular cross-section of the guide body (4), so that a snap-in connection can be produced between the guide body (4) on the one hand and the blind hole (11) with the longish opening on the other and the condylar plate (2) can slide in the direction of the longitudinal axis (18) of the guide body (4) and rotate about the axis of rotation (1).

2. A knee joint endoprosthesis according to claim 1, **characterised in that** the guide body (4) has a circular cylindrical construction.

3. A knee joint endoprosthesis according to claim 1 or 2, **characterised in that** the guide body (4) is flattened on its side that faces the tibial support surface (10).

4. A knee joint endoprosthesis according to claim 1 or 2, **characterised in that** the guide body (4) is flattened on its side that faces the support shells (3).

5. A knee joint endoprosthesis according to any one of claims 1 to 4, **characterised in that** the guide body (4) has a barrel-shaped construction.

6. A knee joint endoprosthesis according to any one of claims 1 to 5, **characterised in that** the lateral regions of the external surface of the guide body (4) have a curved surface, preferably with a common axis of curvature.

7. A knee joint endoprosthesis according to claim 6, **characterised in that** the axis of curvature coincides with the longitudinal axis (18) of the guide body (4).

8. A knee joint endoprosthesis according to any one of claims 1 to 7, **characterised in that** the external surfaces extending parallel to the longitudinal axis (18) of the guide body (4) do not have surfaces that are parallel to one another.

9. A knee joint endoprosthesis according to any one of claims 1 to 8, **characterised in that** the cross-section (17) of the guide body (4) has a circular, preferably a circular segment, construction.

10. A knee joint endoprosthesis according to any one of claims 1 to 9, **characterised in that** the cross-section of the blind hole (11) is so constructed, that by means of an opening (7), the diameter of which is smaller that the diameter of the blind hole (11), on the end face of the blind hole (11) at the anterior end of the condylar plate (2) synovia can penetrate and the blind hole (11) and the guide body (4) interacting with the synovia act as a hydraulic shock-absorber.

11. A knee joint endoprosthesis according to any one of claims 1 to 10, **characterised in that** both the bearing spigot (8) of the guide body (4) and the anchoring part (15) of the tibial support surface (10) are made from a metallic material, preferably from the same metallic material, resulting in a pure metal to metal guiding of the bearing spigot (8).

12. A knee joint endoprosthesis according to any one of claims 1 to 10, **characterised in that** the anchoring part (15) of the tibial support surface (10) is provided with a metal sleeve (14) that is preferably pressed into it, the purpose of which is to accommodate the bearing spigot (8) and that the bearing spigot (8) is also manufactured from a metallic material, preferably form the same metallic material as the metal sleeve (14), resulting in a pure metal to metal guiding.

13. A knee joint endoprosthesis according to any one of claims 1 to 12, **characterised in that** the condylar plate (2) can be displaced in the anterior and posterior directions at the maximum by the length of the blind hole (11) minus the length of the guide body (4).

14. A knee joint endoprosthesis according to any one of claims 1 to 13, **characterised in that** the condylar plate (2) can be displaced in the anterior or posterior directions by the amount of 6 mm < x < 13 mm, preferably 7,5 mm < x < 11,5 mm.

15. A knee joint endoprosthesis according to any one of claims 1 to 14, **characterised in that** the length of the blind hole (11) is longer than the length of the guide body (4) by 0,1 to 3,0 mm, preferably 1 to 2 mm.

16. A knee joint endoprosthesis according to any one of claims 1 to 15, **characterised in that** a play of 0,1 - 0,4 mm, preferably 0,2 - 0,3 mm is provided between the external surface of the guide body (4) extending parallel to the longitudinal axis (18) of the guide body (4) and the inside surface of the blind hole (11) corresponding with it.

17. A knee joint endoprosthesis according to any one of claims 1 to 16, **characterised in that** the underside (19) of the guide body (4) is at a distance of at least 3 mm, preferably at least 4 mm, from the plane of the tibial support surface (10).

18. A method to manufacture a knee joint endoprosthesis according to any one of claims 1 to 17, **characterised in that** the guide body (4) is pressed into the blind hole (11) with snap-in connection in a pre-assembly operation.

## Revendications

1. Endoprothèse de l'articulation du genou comprenant un élément tibial (16) et se composant d'un élément d'ancrage (15) pouvant être fixé dans le tibia et d'une surface d'appui tibiale (10), d'une plaque condylaire (2) disposée sur celle-ci de manière à pouvoir se déplacer, laquelle comprend deux coussinets (3) à courbure concave permettant de loger de manière mobile un élément articulé fémoral et des moyens (2,4,10) assurant un guidage du mouvement de rotation de la plaque condylaire (2) tant en sens rotatoire qu'en sens transversal par rapport à la surface d'appui tibiale (10), la plaque condylaire (2) pouvant tourner sur un axe de rotation (1) s'étendant perpendiculairement par rapport à celle-ci et pouvant être déplacée de manière longitudinale à cet axe, et les moyens (2,4,10) assurant le guidage dudit mouvement de rotation comprenant un corps de guidage (4) rectiligne, en forme de tige, disposé sur la surface d'appui tibiale plane (10) et logé sur celle-ci de manière à tourner sur l'axe de rotation (1) s'étendant perpendiculairement à la surface d'appui tibiale (10),
**caractérisée en ce que**
le corps de guidage (4) est réalisé sous forme de cylindre, qu'il est positionné à une distance définie de la surface d'appui tibiale (10) et qu'il présente un tourillon (8) logé dans l'élément d'ancrage (15) de l'élément tibial (16) de manière à pouvoir tourner, le corps de guidage (4) coopérant avec la plaque condylaire (2) **en ce que** dans la plaque condylaire (2) est prévu un trou borgne (11) avec une ouverture oblongue (12) qui est au moins aussi longue que l'extension longitudinale du corps de guidage (4) et dont la largeur est inférieure au diamètre de la surface de la section transversale du corps de guidage (4), de sorte qu'il se forme une liaison à déclic entre, d'une part, le corps de guidage (4) et, d'autre part, le trou borgne (11) à ouverture oblongue et qu'il est possible à la plaque condylaire (2) de glisser en direction de l'axe longitudinal (18) du corps de guidage (4) et de tourner sur l'axe de rotation (1).

2. Endoprothèse de l'articulation du genou selon la revendication 1, **caractérisée en ce que** le corps de guidage (4) est réalisé en forme de cylindre circulaire.

3. Endoprothèse de l'articulation du genou selon la revendication 1 ou 2, **caractérisée en ce que** le corps de guidage (4) est aplati du côté opposé à la surface d'appui tibiale (10).

4. Endoprothèse de l'articulation du genou selon la revendication 1 ou 2, **caractérisée en ce que** le corps de guidage (4) est aplati du côté opposé aux coussinets (3).

5. Endoprothèse de l'articulation du genou selon l'une des revendications 1 à 4, **caractérisée en ce que** le corps de guidage (4) est réalisé en forme de barillet.

6. Endoprothèse de l'articulation du genou selon l'une des revendications 1 à 5, **caractérisée en ce que** les zones latérales de la surface extérieure du corps de guidage (4) présentent une surface courbe, de préférence avec un axe de courbure commun.

7. Endoprothèse de l'articulation du genou selon la revendication 6, **caractérisée en ce que** l'axe de courbure coïncide avec l'axe longitudinal (18) du corps de guidage (4).

8. Endoprothèse de l'articulation du genou selon l'une des revendications 1 à 7, **caractérisée en ce que** les surfaces extérieures s'étendant parallèlement à l'axe longitudinal (18) du corps de guidage (4) ne présentent pas de surfaces parallèles entre elles.

9. Endoprothèse de l'articulation du genou selon l'une des revendications 1 à 8, **caractérisée en ce que** la section transversale (17) du corps de guidage (4) est réalisée de forme circulaire, de préférence en forme de segment de cercle.

10. Endoprothèse de l'articulation du genou selon l'une des revendications 1 à 9, **caractérisée en ce que** le trou borgne (11) présente une section transversale formée de telle sorte qu'au niveau de la face frontale du trou borgne (11) à l'extrémité antérieure de la plaque condylaire (2), de la synovie peut pénétrer, par une ouverture (7) dont le diamètre est inférieur au diamètre du trou borgne (11), et **en ce que** le trou borgne (11) et le corps de guidage (4) coopèrent avec la synovie pour former un amortisseur hydraulique.

11. Endoprothèse de l'articulation du genou selon l'une des revendications 1 à 10, **caractérisée en ce que** et le tourillon (8) du corps de guidage (4) et l'élément d'ancrage (15) de la surface d'appui tibiale (10) sont réalisés en un matériau métallique, de préférence le même matériau métallique, de sorte à obtenir un guidage du type métal-métal du tourillon (8).

12. Endoprothèse de l'articulation du genou selon l'une des revendications 1 à 10, **caractérisée en ce que** l'élément d'ancrage (15) de la surface d'appui tibiale (10) est pourvu d'une douille métallique (14), de préférence insérée dans celui-ci par pression, laquelle est destinée à recevoir le tourillon (8), et **en ce que** le tourillon (8) est également réalisé en un matériau métallique, de préférence le même matériau métallique que la douille métallique (14), de sorte à obtenir un guidage du type métal-métal.

13. Endoprothèse de l'articulation du genou selon l'une des revendications 1 à 12, **caractérisée en ce que** la plaque condylaire (2) est déplaçable en direction antérieure ou en direction postérieure au maximum sur une longueur correspondant à la longueur du trou borgne (11) moins la longueur du corps de guidage (4).

14. Endoprothèse de l'articulation du genou selon l'une des revendications 1 à 13, **caractérisée en ce que** la plaque condylaire (2) est déplaçable en direction antérieure ou en direction postérieure d'une distance 6 mm < x < 13 mm, de préférence 7,5 mm < x < 11,5 mm.

15. Endoprothèse de l'articulation du genou selon l'une des revendications 1 à 14, **caractérisée en ce que** la longueur du trou borgne (11) est supérieure de 0,1 à 3,0 mm, de préférence de 1 à 2 mm, à la longueur du corps de guidage (4).

16. Endoprothèse de l'articulation du genou selon l'une des revendications 1 à 15, **caractérisée en ce qu'**un jeu de 0,1 à 0,4 mm, de préférence de 0,2 à 0,3 mm, est prévu entre la surface extérieure du corps de guidage (4) s'étendant parallèlement à l'axe longitudinal (18) dudit corps de guidage (4) et la surface intérieure correspondante du trou borgne (11).

17. Endoprothèse de l'articulation du genou selon l'une des revendications 1 à 16, **caractérisée en ce que** la face inférieure (19) du corps de guidage (4) présente une distance d'au moins 3 mm, de préférence d'au moins 4 mm, par rapport au plan de la surface d'appui tibiale (10).

18. Procédé permettant de fabriquer une endoprothèse de l'articulation du genou selon l'une des revendications 1 à 17, **caractérisé en ce que** le corps de guidage (4) est, lors d'un prémontage, inséré par pression dans le trou borgne (11) grâce à la liaison à déclic.
